(19) Europäisches Patentamt
European Patent Office
Office européen des brevets

(11) **EP 2 036 489 A2**

(12) **EUROPEAN PATENT APPLICATION**

(43) Date of publication:
**18.03.2009 Bulletin 2009/12**

(51) Int Cl.:
***A61B 5/00*** (2006.01)

(21) Application number: **08016007.0**

(22) Date of filing: **11.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **12.09.2007 JP 2007236439**

(71) Applicant: **Canon Kabushiki Kaisha Tokyo (JP)**

(72) Inventors:
• **Yoshida, Hirofumi**
  **Tokyo (JP)**
• **Nishihara, Hiroshi**
  **Tokyo (JP)**
• **Masumura, Takahiro**
  **Tokyo (JP)**

(74) Representative: **Weser, Wolfgang**
  **Weser & Kollegen**
  **Patentanwälte**
  **Radeckestrasse 43**
  **81245 München (DE)**

(54) **Measurement apparatus**

(57)    A measurement apparatus (100) includes an arithmetic processing means (14) which calculates the ratio of both collagen and lipid relative to the whole of a plurality of ingredients including collagen and lipid from a measurement result of the measurement means and the absorption coefficients of each ingredient, and determines the relationship of the fitting coefficients ($\mu_a$) of the lipid and collagen and the state of biological tissue, and then determines the state of biological tissue of the specimen from the ratio of collagen and the ratio of lipid which were calculated, and a display unit (17) which displays a result of processing by the arithmetic processing means. The measurement means uses a light having a predetermined wavelength within the wavelength range between 600nm and 700nm and at least two types of light having different wavelengths within the wavelength range between 730nm to 760nm as the plurality of types of light.

FIG.1

**Description**

BACKGROUND OF THE INVENTION

Field of the Invention

The present invention relates to a measurement apparatus configured to measure a spectroscopic characteristic in a specimen.

Description of the Related Art

A conventional measurement apparatus as used for mammography that utilizes the light for a measurement measures a metabolism and a related spectroscopic characteristic in a specimen (scattering medium) and creates an image of a spatial distribution of a spectroscopic characteristic. The spectroscopic characteristic includes an absorption (spectroscopic) characteristic and a scattering (spectroscopic) characteristic. It is desirable for the medical diagnosis to establish the technology of easily determining a state of a biological tissue based on a spectroscopic characteristic. A "state of a biological tissue," as used herein, means a normal tissue, a benign tumor, a malignant tumor, etc. Conventionally, there are proposed a number of tumor identifying methods and tumor classifying methods.

Japanese Patent No. 3,107,914 and "Near-Infrared Characterization of Breast Tumors In Vivo using Spectrally-Constrained Reconstruction," Dartmouth, Pogue, 2005 disclose a tumor identifying method that uses the near-infrared light and obtains deoxygenated hemoglobin, oxygenated hemoglobin and a ratio of the oxygenated hemoglobin to a total amount (oxygen saturation). This method, which will be referred to as a "hemoglobin method" hereinafter, utilizes the fact that a tumor has a larger total amount of deoxygenated hemoglobin and oxygenated hemoglobin and lower oxygen saturation than a normal tissue.

"Diagnosing breast cancer by using Raman spectroscopy," MIT, Haka, Proc Natl Acad Sci USA, 2005, discloses a method of extracting a micro tissue from a specimen, of detecting collagen using the Raman spectroscopy, and of detecting a tumor using detected collagen. Since a ratio of collagen in a tumor to a normal tissue is higher than the oxygen saturation of hemoglobin, the method using collagen is advantageous in precisely detecting a tumor. Furthermore, this literature reports that the state of the tumor can be determined by investigating a ratio of each of lipid and collagen to the whole.

"Absorption properties of breast: the contribution of collagen," ULTRAS-CNR-INFM and IFN-CNR, Politecnicodi Milano, 2006, obtains an absorption coefficient of a specimen using the near-infrared light having a wide wavelength range, and a ratio of each of the ingredients including collagen through fitting.

"Assessment of collagen absorption and related potential diagnostic applications," SPIE-OSAVol.662966290D-1 obtains a ratio of the ingredient in a specimen through fitting using wavelengths of 637 nm, 680 nm, 785 nm, 905 nm, 933 nm, and 1,060 nm. This literature precisely estimates hemoglobin using collagen as a parameter, and improves a tumor detecting precision. Furthermore, according to this literature, an area with a high mammographic density is likely to become a tumor, which is said to have a large amount of collagen. Since a main ingredient of a stroma is collagen and a structure and structural change of a stroma relate to the condition of both benign and malignant pathological changes, collagen plays a role of breast cancer carcinogenesis in the early stage.

However, the hemoglobin method has a low precision of characterizing tumor, and the Raman spectroscopy places a burden on a specimen since the specimen must be cut open in order to extract a tissue.

The method disclosed in "Absorption properties of breast by the contribution of collagen," supra, is impractical since it requires time-consuming measurements by using the various luminous fluxes of a wide wavelength range. Further, "Assessment of collagen absorption and related potential diagnostic applications," supra estimates hemoglobin using collagen as a parameter and determines a state of a biological tissue based on the estimated hemoglobin, rather than determining the state of the biological tissue directly based on collagen.

Thus, the conventional measurement methods cannot precisely and easily determine a state of a biological tissue in a specimen (without time-consuming measurements or a burden on the specimen by making incisions and the like).

SUMMARY OF THE INVENTION

The present invention provides a measurement apparatus configured to precisely and easily measure a state of a biological tissue of a specimen.

The present invention in its first aspect provides a measurement apparatus as specified in claims 1 to 9.

Further detailed objects and other characteristics of the present invention will become apparent by the preferred embodiments described below referring to accompanying drawings which follow.

BRIEF DESCRIPTION OF THE DRAWINGS

FIG. 1 is block diagram of a measurement apparatus according to a first embodiment of the present invention.

FIG. 2 is schematic sectional view of a structure in which the measurement apparatus shown in FIG. 1 is applied to detect a breast cancer.

FIG. 3 is a flowchart for explaining an operation of the measurement apparatus shown in FIG. 1.

FIG. 4 shows absorption spectra of collagen, lipid, water, deoxygenated hemoglobin, and oxygenated hemoglobin which are main ingredients in a biological tissue.

FIG. 5 is a graph that compares a measured value relating to an absorption spectrum of collagen with a value described in "Absorption properties of breast: the contribution of collagen."

FIG. 6 shows a mapping result of a ratio of collagen.

FIG. 7 shows mapping results of lipid and water.

FIG. 8 is a graph which shows a relationship between a fitting coefficient of lipid and a fitting coefficient of collagen.

FIG. 9 is a mapping result of the state shown in FIG. 8.

FIG. 10 shows mapping results of ratios of deoxygenated hemoglobin and oxygenated hemoglobin.

FIG. 11 is a mapping result of each value of oxygen saturation.

FIG. 12 is mapping results of simultaneous expressions of a distribution of a state of a biological tissue and a distribution of oxygen saturation.

FIG. 13 is a block diagram of a measurement apparatus according to a second embodiment of the present invention.

FIG. 14 is a flowchart of an operation of the measurement apparatus shown in FIG. 13.

FIG. 15 is a block diagram of a measurement apparatus according to a third embodiment of the present invention.

FIG. 16 is flowchart of an operation of the measurement apparatus shown in FIG. 15.

DESCRIPTION OF THE EMBODIMENTS

A measurement apparatus according to the embodiment of the present invention includes a measurement unit (a measurement means), an arithmetic processing unit (an arithmetic processing means), and a display unit.

The measurement unit irradiates into a specimen (scattering medium) as an absorption-scattering body, a plurality of types of luminous fluxes having different central wavelengths in a range of 600 nm to 1,000 nm, and measures a spectroscopic characteristic in a specimen. In this way, the measurement unit uses a wavelength range referred as an optical window of the near-infrared light, and requires no incisions of the specimen unlike "Diagnosing breast cancer by using Ramanspectroscopy," supra.

As will be explained later, the measurement unit can apply any of the following methods of measurement: Diffuse Optical Tomography ("DOT"), Acousto-Optical tomography ("AOT"), and Photo-Acoustic Tomography ("PAT"). DOT introduces the near-infrared light into a specimen, and detects the diffused light. The incident light may be the light from a light source whose intensity is modulated, or the incident light may use the pulsed light. AOT irradiates the coherent light and focused ultrasound into a measurement site, and detects the modulated light by a light detecting device (a light detector) using a light modulation effect (or acousto-optical effect) in an ultrasound focusing area. PAT utilizes a difference in absorption factor of the light energy between a measurement site, such as a tumor, and another tissue, and receives through a transducer an elastic wave (or acousto-optical signal) that occurs as a result of that the measurement site absorbs the irradiated light energy and instantly swells.

The arithmetic processing unit calculates a ratio of each of collagen and lipid to the entire amount of a plurality of ingredients including collagen and lipid, based on a measurement result of the measurement unit and an absorption coefficient in each ingredient. Next, the arithmetic processing unit determines a state of a biological tissue in the specimen based on a relationship between a fitting coefficient of each of lipid and collagen and a state of a biological tissue, a calculated ratio of collagen, and a calculated ratio of lipid. The following embodiment determines that a biological tissue of the specimen is in any one of states of a normal tissue, a fiber tumor, a changing fibroadenoma, and a infiltrating carcinoma, but the present invention does not limit the type or the number of states of the biological tissue in the specimen.

The display unit displays a processing result of the arithmetic processing unit.

A description will now be given of a measurement apparatus according to embodiments.

FIRST EMBODIMENT

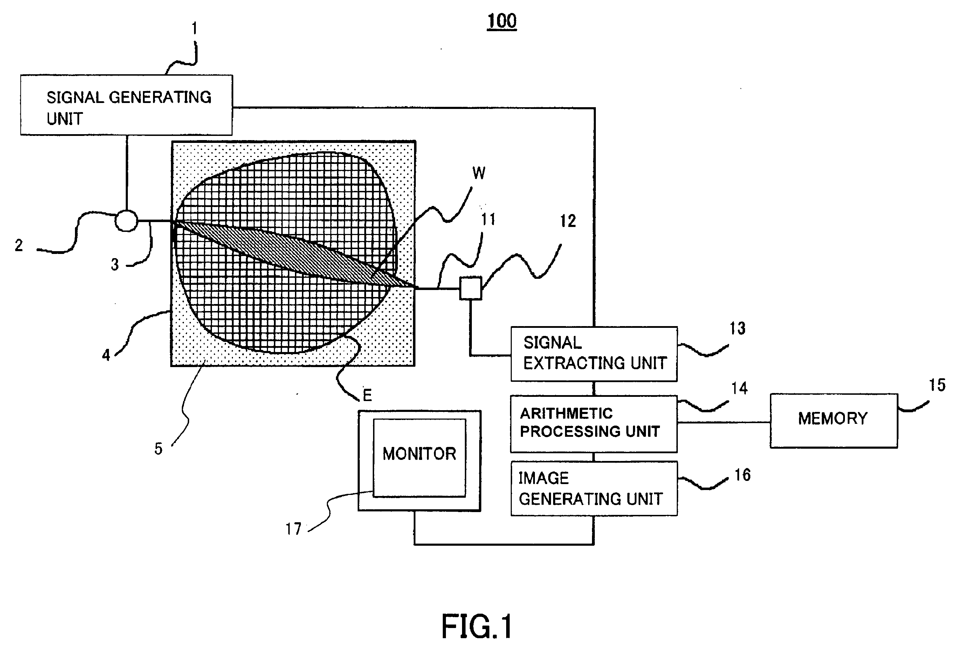

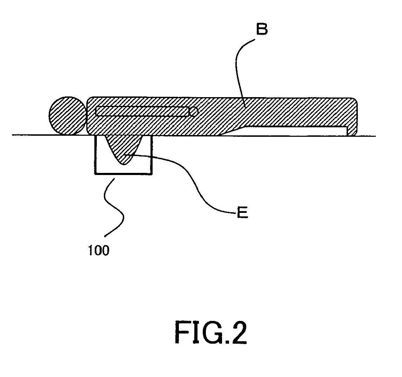

FIG. 1 is a block diagram of a measurement apparatus 100 according to a first embodiment of the present invention. A specimen E is an object to be measured as an absorption-scattering body, and specifically has a biological tissue such as a breast. FIG. 2 is a schematic sectional view of a configuration in which the measurement apparatus 100 is applied to detect a breast cancer in the specimen (breast) E of an examinee B.

The measurement unit in the measurement apparatus 100 includes a signal generating unit 1, a light source 2, optical fibers 3 and 11, a measurement vessel 4, a light detecting device 12, and a signal extracting unit 13.

The specimen E is housed in the measurement vessel 4. A uniform medium (matching material 5) having a known characteristic is filled in a space between the specimen E and the measurement vessel 4, and considered to have substantially the same refractive index of the light, scattering coefficient, and acoustic characteristic of the ultrasound as those of the specimen E. The light source 2 utilizes a semiconductor laser, and emits the intensity-modulated light at a frequency $f_1$ via a signal generating unit (sine wave transmitter) 1. In general, the light may be modulated with a sine wave having several tens to hundreds of MHz in a bioinstrumentation. Via the optical fiber 3, the light from the light source 2 enters a side surface of the measurement vessel 4. The modulated light that has entered the vessel propagates in the specimen E as an energy density wave (diffusion photon density wave) W of a modulation frequency $f_1$ as derived from the light diffusion theory. Via the optical fiber 11, the light detecting device 12, such as a PMT (Photo Multiplier) and an APD (Avalanche Photo Diode), detects the diffusion photon density wave W as signal light that transmits at the modulation frequency $f_1$. The signal extracting unit 13 extracts required information from the diffusion photon density wave W.

Based on the information of the spectroscopic characteristic extracted by the signal extracting unit 13, the arithmetic processing unit 14 calculates an absorption coefficient and a scattering coefficient of the specimen E, and calculates a ratio of each ingredient in specimen E based on the absorption coefficient and the scattering coefficient. The arithmetic processing unit 14 stores the calculated absorption coefficient and scattering coefficient and the ratio of the ingredient in the memory 15. An image generating unit 16 maps the stored value. A display unit 17 displays distributions of three-dimensional absorption and scattering coefficients, and a ratio of each ingredient.

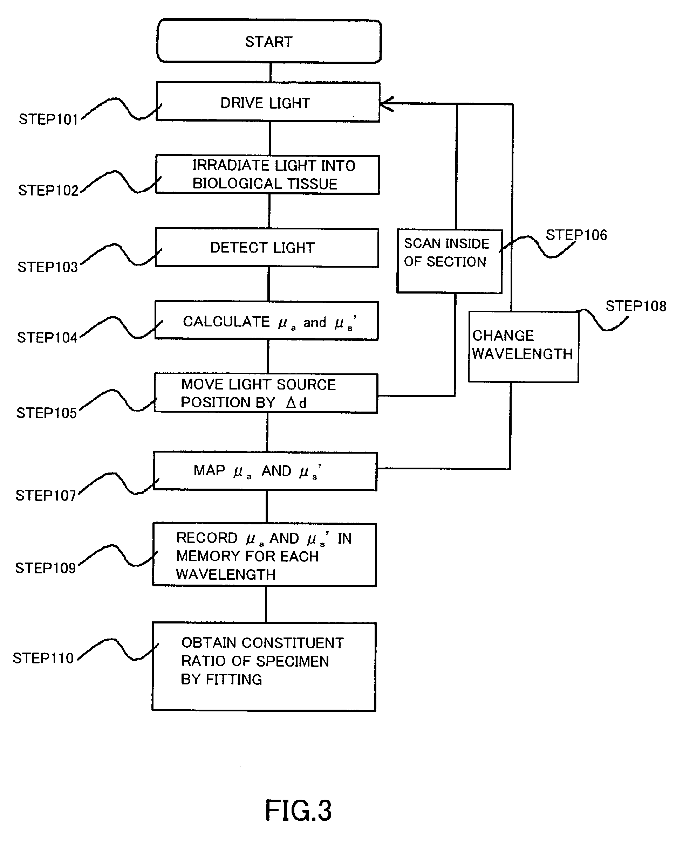

FIG. 3 is a flowchart for explaining an operation of the measurement apparatus 100 to obtain a tomographic image of one section of the specimen E. Initially, in the step 101, the signal generating unit 1 modulates the intensity of the light source 2 with the frequency f1 (several tens to hundreds of MHz) and drives it as a light source having a certain wavelength. In the step 102, the light source 2 introduces the light into the specimen E via the optical fiber 3 from a certain position. Next, in the step 103, the light detecting device 12 measures an amplitude $I_{AC}$ (r, t) and a phase $\Phi$(r, t) of the modulated light via the optical fiber 11. In general, an optical path that propagates the diffusion photon density wave W has a spindle shape, and the obtained absorption coefficient and scattering coefficient are average values in the optical path.

The step 104 obtains the absorption coefficient $\mu_a$ and equivalent scattering coefficient $\mu_s$' based on the obtained amplitude and phase. This embodiment uses an approximation solution derived from a light diffusion equation, but the present invention is applicable to more rigorous solutions.

An optical power I (r, t) [photon/ sec·mm2] is given by the following equation where t is time, and r is a distance from a point light source in a uniform absorption-scattering body:

EQUATION 1

$$I = I_{DC} + I_{AC}\exp[-i(\Phi+\varepsilon)]$$

EQUATION 2

$$I_{AC} = \frac{A}{4\pi Dr}\exp\left[-r\left(\frac{v^2\mu_a^2+\omega^2}{v^2D^2}\right)^{1/4}\cos\left[\frac{1}{2}\tan^{-1}\left(\frac{\omega}{v\mu_a}\right)\right]\right]$$

EQUATION 3

$$\Phi = r\left(\frac{v^2\mu_a^2+\omega^2}{v^2D^2}\right)^{1/4}\sin\left[\frac{1}{2}\tan^{-1}\left(\frac{\omega}{v\mu_a}\right)\right]$$

EQUATION 4

$$D = 1 / 3\mu_s'$$

$I_{DC}$ [photon/ sec·mm$^2$] is a biased component of the detected light intensity. $\varepsilon$ is an arbitrary phase term. A [photon/ sec] is the number of incident photons in a second light source. D [mm] is a diffusion coefficient. $\nu$ [mm/sec] is the light speed in the absorption-scattering body. $\mu_a$ [mm$^{-1}$] is an absorption coefficient. $\mu_{s,}$ [mm$^{-1}$] is an equivalent scattering coefficient. $\omega$ [rad/sec] is an angular frequency of a modulated wave which has been modulated with ultrasound (when the modulation frequency is f, $\omega$ = 2nf).

Once the amplitude $I_{AC}$ (r, t) and phase $\Phi$(r, t) are measured, the absorption coefficient $\mu_a$ and equivalent scattering coefficient $\mu_s'$ can be calculated from Equations 2 and 3.

Next, the step 105 shifts a position of the optical fiber 3 relative to the specimen E by $\Delta$d, and detects the diffusion photon density wave W by the light detecting device 12. Alternatively, a plurality of optical fibers 3 may be previously installed at positions shifted by $\Delta$d and the diffusion photon density wave W may be detected by the light detecting device 12. This shift $\Delta$d causes a shift of the optical path of the diffusion photon density wave relative to the specimen E, and the absorption coefficient and the scattering coefficient at the shifted position can be calculated. A tomographic image on one section can be obtained when the above flow is repeated and the step 107 maps the absorption-scattering characteristic of the specimen E. Similarly, the tomographic image on one section of the specimen E can be also obtained by shifting one or both positions of the optical fiber 11 and the specimen E. By scanning this section in the vertical direction of the paper plane, three-dimensional absorption-scattering information of the specimen E can finally be obtained.

Next, the step 108 changes a wavelength of the light source. For this method, a plurality of light sources having different wavelengths may introduce the luminous fluxes from separate locations or from bundled fibers at one location. Alternatively, a white light source may be prepared, and the light from the white light may be separated by a diffraction grating, or the light having only a specific wavelength may be selected by a wavelength filter. The modulated light having another wavelength is introduced into the specimen E by any one of these methods, and the operation of the above steps 101-107 is repeated. The step 109 sequentially stores the measured amplitude $I_{AC}$ $(r_i, \theta_j)$ and phase $\Phi(r_i, \theta_j)$, and calculated $\mu_a$ and $\mu_s'$ in the memory 15 for each wavelength. They are uniquely calculated when the number of the used wavelengths is set equal to the number of ingredients of the specimen E. However, a constituent ratio is erroneously estimated when measurement values of the measured $\mu_a$ and $\mu_s'$ shift from true values for some errors (e.g., due to a output shift of an output of light source 2, due to a positional shift between the light source 2 and light detecting device 12, due to a shift caused by disturbance light). Accordingly, plural luminous fluxes having wavelengths more than the number of ingredients are introduced.

Next, in the step 110, the arithmetic processing unit 14 executes fitting by the following method and finds a constituent ratio of the specimen, particularly a ratio of each of collagen, lipid, and water.

In general, a measurement wavelength is changed according to a target ingredient. This is because an absorption coefficient of each ingredient shows an absorption coefficient spectrum (which will be referred to as an "absorption spectrum" hereinafter) unique to each wavelength, and the estimation precision improves through a measurement that uses a characteristic wavelength for each ingredient.

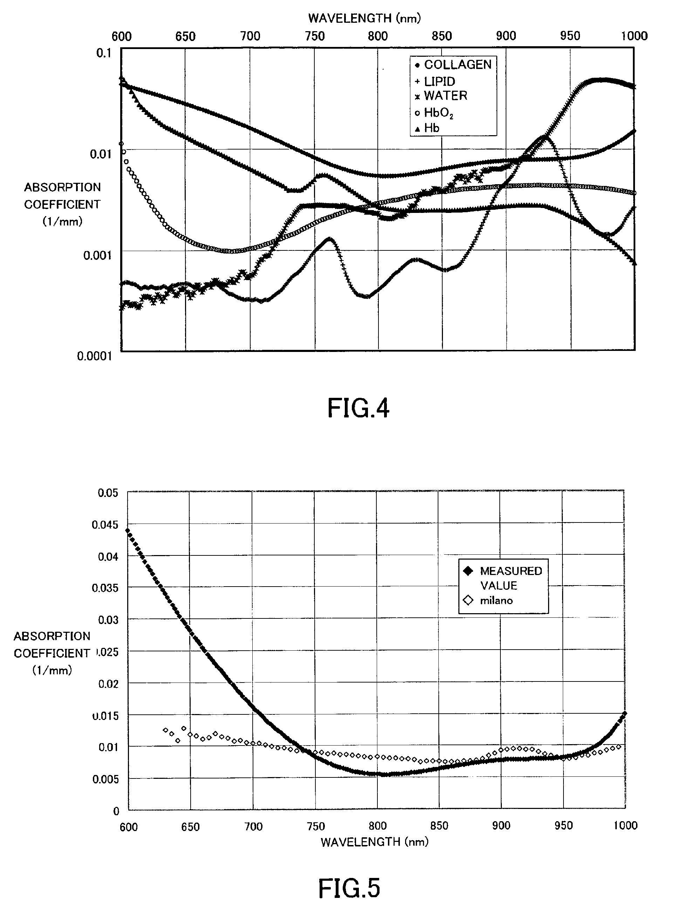

FIG. 4 is absorption spectra of collagen, lipid, water, deoxygenated hemoglobin, and oxygenated hemoglobin, which are the main ingredients in a biological tissue. For example, the absorption spectra of deoxygenated hemoglobin and oxygenated hemoglobin intersect each other at about 800 nm. Deoxygenated hemoglobin has a sharp peak at about 760 nm (or a point having a higher absorption coefficient than the neighboring wavelengths), whereas oxygenated hemoglobin has a modest peak at about 920 nm. Both are likely to become larger as a wavelength becomes shorter, and both are likely to become smaller as a wavelength becomes longer.

The conventional hemoglobin method attempts to identify both utilizing these characteristics, and mainly uses a wavelength between 600 nm and 700nm and a wavelength around 800 nm. In order to identify water and lipid in addition to deoxygenated hemoglobin and oxygenated hemoglobin, a wavelength of 900 nm or greater is used, because the above characteristic peak exists at a wavelength of 900 nm or greater. Apparently, "Diagnosing breast cancer by using Raman spectroscopy," supra uses wavelengths of 661 nm, 761 nm, 785 nm, 808 nm, 826 nm, and 849 nm for this reason.

On the other hand, use of the wavelength between 600 nm and 700 nm, the wavelength around 800 nm, and the wavelength of 900 nm or greater cannot provide a precise estimation to identify collagen. This is because the absorption spectrum of collagen shown in FIG. 3 does not have a peak in this wavelength range.

Nevertheless, the following characteristic wavelength can be found by comparing the collagen's absorption spectrum with the absorption spectra of the other ingredients. Firstly, collagen has a higher absorption coefficient in a range between 600 nm and 700 nm than other absorption spectra. Secondly, only collagen has an absorption coefficient with a negative gradient (in which the absorption coefficient decreases as the wavelength increases) in a range between 730 nm and 760 nm when compared to other absorption spectra.

FIG. 5 is a graph which compares an actual measurement value relating to an absorption spectrum of collagen with a value described in "Absorption properties of breast: the contribution of collagen," supra. Referring to FIG. 5, it is understood that they are similar enough to identify collagen in the above two points although the absolute values are significantly different. Furthermore, since water has a high absorption coefficient near 1,000 nm as understood from FIG. 4, water absorption becomes too remarkable to measure a deep portion in the biological tissue when the specimen E contains a large amount of water.

In order to precisely estimate a constituent ratio of only collagen, it is effective to use a luminous flux having a wavelength between 600 nm and 700 nm, and at least two luminous fluxes having different wavelengths in a wavelength range between 730 nm and 760 nm. In general, a measurement using a wavelength range in which a target ingredient has a high absorption coefficient is likely to improve an estimation precision due to a high sensitivity of the ingredient, and thus a wavelength in a range between 600 nm and 700 nm is selected. Since a light amount emitted from the specimen decreases as an absorption coefficient increases, it is noted that the measurement precision lowers when the absorption coefficient becomes excessively high. Further, since at least two wavelengths are needed to check a gradient of the second characteristic, at least two luminous fluxes having different wavelengths in a range between 730 nm and 760 nm are used. When the measurement uses these wavelengths, the memory 15 can store three types of $\mu_a$ where $\mu_{a\_\lambda}$ represents data of an absorption coefficient $\mu_a$ of a certain mapped portion for each wavelength:

EQUATION 5

$$\begin{pmatrix} \mu_{a-640} \\ \mu_{a-730} \\ \mu_{a-760} \end{pmatrix}$$

$C_{content}$ as a constituent ratio can be expressed as follows:

EQUATION 6

$$\begin{pmatrix} c_{collagen} \\ c_{HbO_2} \\ c_{Hb} \\ c_{lipid} \\ c_{water} \end{pmatrix}.$$

An absorption coefficient can be obtained by multiplying a distribution of absorption coefficients $\mu_{a\_\lambda\_content}$ of each known ingredient shown in FIG. 4 by Equation 6:

EQUATION 7

$$\begin{pmatrix} \mu_{a-640\text{-}collagen} & \mu_{a-640\text{-}HbO_2} & \mu_{a-640\text{-}Hb} & \mu_{a-640\text{-}lipid} & \mu_{a-640\text{-}water} \\ \mu_{a-730\text{-}collagen} & \mu_{a-730\text{-}HbO_2} & \mu_{a-730\text{-}Hb} & \mu_{a-730\text{-}lipid} & \mu_{a-730\text{-}water} \\ \mu_{a-760\text{-}collagen} & \mu_{a-760\text{-}HbO_2} & \mu_{a-760\text{-}Hb} & \mu_{a-760\text{-}lipid} & \mu_{a-760\text{-}water} \end{pmatrix} \bullet \begin{pmatrix} C_{collagen} \\ C_{HbO_2} \\ C_{Hb} \\ C_{lipid} \\ C_{water} \end{pmatrix}$$

All constituent ratios can be obtained in the measurement by fitting $C_{content}$ in Equation 7 to Equation 5 using a fitting method, such as a least-squares method. In the above measurement wavelength range, the obtained constituent ratios other than that of collagen are likely to have errors, because the used wavelengths are characteristic only to collagen as described above. On the other hand, only for collagen, the calculated constituent ratio is more precise and an amount of collagen or a ratio of collagen to the total amount can be obtained.

As described above, after an amount of collagen or its ratio to the total amount for a portion on a section of the specimen E is obtained, a similar flow follows with a mapped absorption coefficient $\mu_a$ so as to map an amount of collagen or its ratio to the total amount on the section of the specimen E. By scanning this section, distributions of three-dimensional absorption-scattering information of the specimen E and an amount of collagen or its ratio to the total amount can be finally obtained.

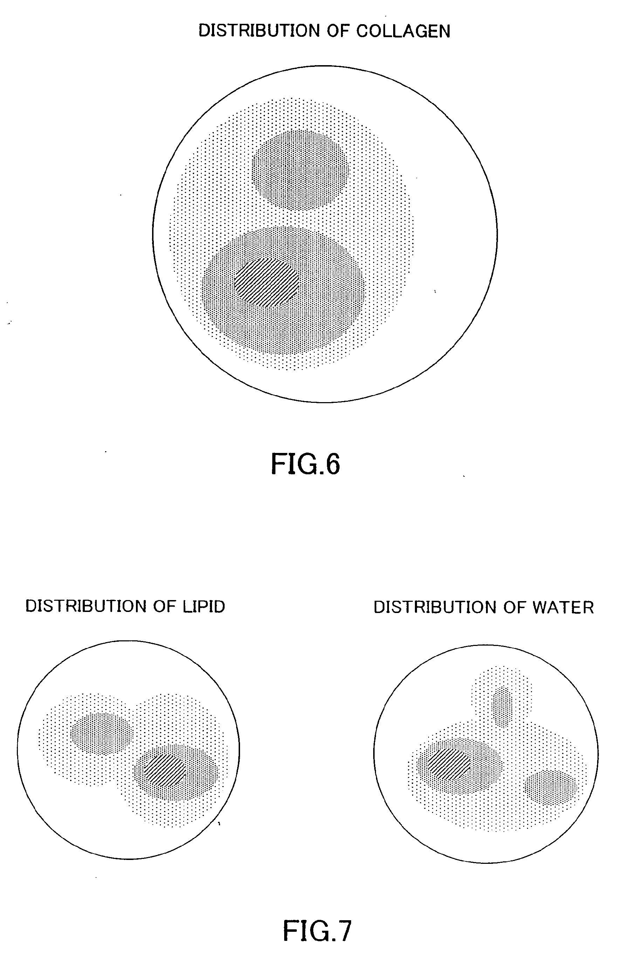

FIG. 6 illustrates a mapping example of a ratio of collagen, and correlates a value of $C_{collagen}$ calculated at each site in the specimen with a corresponding position, expressing a degree of the value by a color depth. This expression provides quick visual confirmation of a location of a high collagen concentration and a location of a low collagen concentration.

Use of two wavelengths in a range between 900 nm and 1,000 nm in addition to the above wavelengths, amounts of collagen, lipid, and water or their ratios to the total amount can be precisely obtained, because lipid and water have high absorption coefficients at wavelengths of 900 nm or greater as shown in FIG. 4. For example, the measurement may use additional wavelengths of 910 nm and 970 nm, or use 640 nm, 730 nm, 760 nm, 910 nm and 970 nm. When the measurement uses the above five wavelengths, the memory 15 can store five types of $\mu_a$ where $\mu_{a\_\lambda}$ represents data of an absorption coefficient $\mu_a$ of a certain mapped portion for each wavelength:

EQUATION 8

$$\begin{pmatrix} \mu_{a-640} \\ \mu_{a-730} \\ \mu_{a-760} \\ \mu_{a-910} \\ \mu_{a-950} \end{pmatrix}$$

$C_{content}$ as a constituent ratio is expressed as follows:
EQUATION 9

$$\begin{pmatrix} C_{collagen} \\ C_{HbO_2} \\ C_{Hb} \\ C_{lipid} \\ C_{water} \end{pmatrix}$$

A value of an absorption coefficient can be obtained by multiplying a distribution of the absorption coefficients $\mu_{a\_\lambda\_content}$ of each known ingredient shown in FIG. 3 by Equation 9:

EQUATION 10

$$\begin{pmatrix} \mu_{a-640-collagen} & \mu_{a-640-HbO_2} & \mu_{a-640-Hb} & \mu_{a-640-lipid} & \mu_{a-640-water} \\ \mu_{a-730-collagen} & \mu_{a-730-HbO_2} & \mu_{a-730-Hb} & \mu_{a-730-lipid} & \mu_{a-730-water} \\ \mu_{a-760-collagen} & \mu_{a-760-HbO_2} & \mu_{a-760-Hb} & \mu_{a-760-lipid} & \mu_{a-760-water} \\ \mu_{a-910-collagen} & \mu_{a-910-HbO_2} & \mu_{a-910-Hb} & \mu_{a-910-lipid} & \mu_{a-910-water} \\ \mu_{a-970-collagen} & \mu_{a-970-HbO_2} & \mu_{a-970-Hb} & \mu_{a-970-lipid} & \mu_{a-970-water} \end{pmatrix} \bullet \begin{pmatrix} C_{collagen} \\ C_{HbO_2} \\ C_{Hb} \\ C_{lipid} \\ C_{water} \end{pmatrix}$$

Constituent ratios of collagen, lipid, and water can be precisely obtained by fitting $C_{content}$ in Equation 10 to Equation 8 using a fitting method, such as a least-squares method, and amounts of collagen, lipid, and water or their ratios to the total amount can be obtained.

As described above, after amounts of collagen, lipid, and water or their ratios to the total amount for a portion on a section of the specimen E is obtained, a similar flow follows with a mapped absorption coefficient $\mu_a$ so as to map amounts of collagen, lipid, and water or their ratios to the total amount on one section of the specimen E. By scanning this section, distributions of three-dimensional absorption-scattering information of the specimen E and the amounts of collagen, lipid, and water or their ratios to the total amount can be finally obtained.

FIG. 7 illustrates a mapping example of ratios of lipid and water, and correlates values of the $C_{lipid}$ and $C_{water}$ calculated at each site in the specimen E with corresponding positions, expressing a degree of the value by a color depth. This expression provides quick visual confirmations of a location of a high lipid or water concentration and a location of a low lipid or wafer concentration.

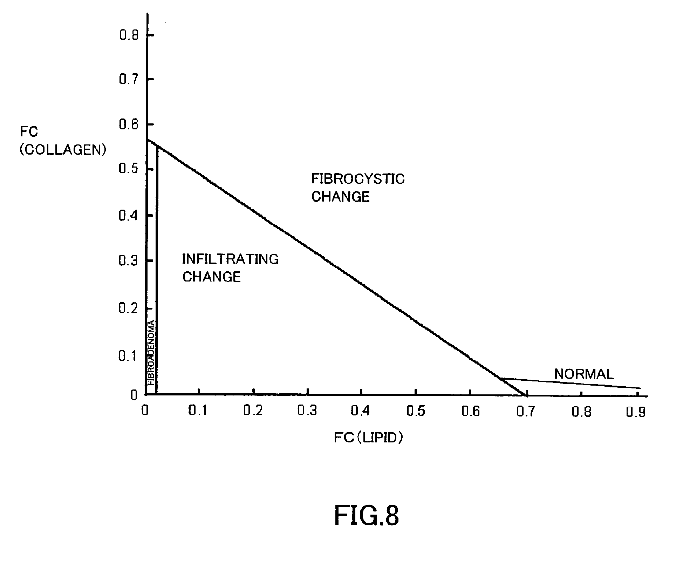

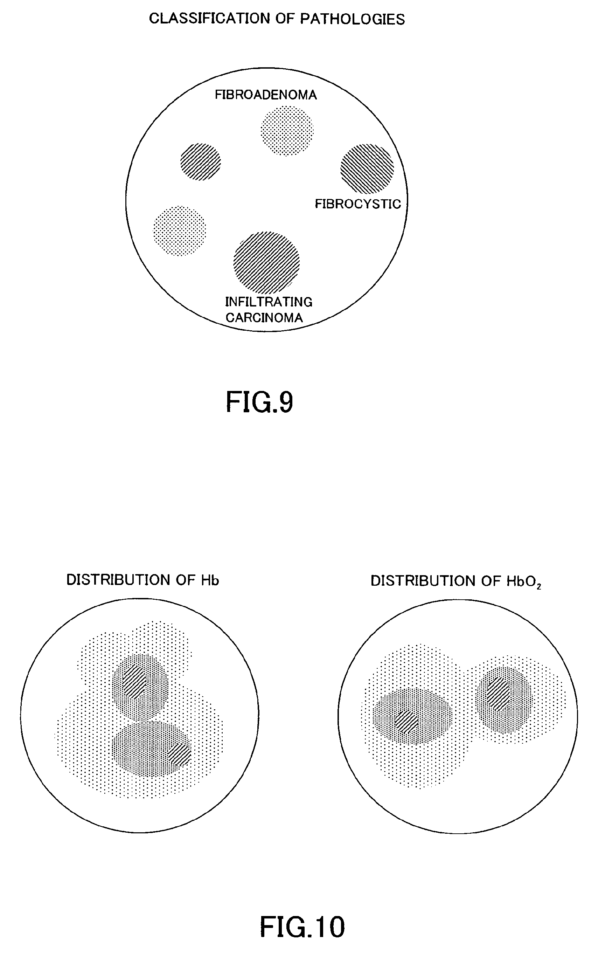

The measurement apparatus 100 can be used to confirm pathologies by estimating the ratios of collagen and lipid. FIG. 8 is a graph of a state of a biological tissue where an abscissa axis denotes a fitting coefficient (Fit Coefficient: FC) of lipid and an ordinate axis denotes a fitting coefficient of collagen. FC is a coefficient of fitting, and expressed as a value of a ratio to the total amount that is set to 1. A state of a biological tissue of the specimen E is determined based on a combination of the FC of lipid and the FC of collagen shown in FIG. 8. Once the FC of lipid and the FC of collagen of the measurement site are estimated, the state of the biological tissue is determined by plotting the value in FIG. 8. The biological tissue is classified into any one of a normal tissue, a fibrocystic change, a fibroadenoma and an infiltrating carcinoma the biological tissue. FIG. 9 is a mapping result of each state. When the display unit 17 displays a partial or entire state of the specimen E shown in FIG. 9, a position and a type of a tumor of the specimen E can be confirmed.

"Diagnosing breast cancer by using Raman spectroscopy," supra, discloses that a tumor has an amount of collagen 4 times as large and an amount of lipid 1/4 times as large as a normal tissue. A measurement site is likely to be a tumor when it has an amount of collagen 4 times as large as and an amount of lipid 1/4 times as large as a surrounding tissue, the same distribution in the past, or the other of a pair in the specimen in which the left and right sides have the same structure, such as a lung and a breast. A quadruple amount change between a tumor and a normal tissue is much larger than an about 1.7 times amount change in the hemoglobin method, and thus has an advantage of a high measurement precision.

Use of two wavelengths in a range between 760 nm and 850 nm to estimate only collagen provides precise estimations of amounts of collagen, deoxygenated hemoglobin, and oxygenated hemoglobin or their ratios to the total amount. As shown in FIG. 4, this is because a cross point between deoxygenated hemoglobin and oxygenated hemoglobin exists near 800 nm, and thus it is easy to detect their changes by using a wavelength near 800 nm.

For example, the measurement may add two wavelengths of 800 nm and 850 nm, or use 640 nm, 730 nm, 760 nm, 800 nm and 850 nm. When the measurement uses the above five wavelengths, the memory 15 can store five types of $\mu_a$ shown in Equation 11 where $\mu_{a\_\lambda}$ represents data of an absorption coefficient $\mu_a$ of a certain mapped section for each wavelength. $C_{content}$ as a constituent ratio can be expressed as in Equation 12:

EQUATION 11

$$\begin{pmatrix} \mu_{a-640} \\ \mu_{a-730} \\ \mu_{a-760} \\ \mu_{a-800} \\ \mu_{a-850} \end{pmatrix}$$

EQUATION 12

$$\begin{pmatrix} C_{collagen} \\ C_{HbO_2} \\ C_{Hb} \\ C_{lipid} \\ C_{water} \end{pmatrix}$$

A value of an absorption coefficient can be obtained by multiplying a distribution of an absorption coefficient $\mu_{a\_\lambda\_content}$ of each known ingredient shown in FIG. 3 by Equation 12:

EQUATION 13

$$\begin{pmatrix} \mu_{a-640-collagen} & \mu_{a-640-HbO_2} & \mu_{a-640-Hb} & \mu_{a-640-lipid} & \mu_{a-640-water} \\ \mu_{a-730-collagen} & \mu_{a-730-HbO_2} & \mu_{a-730-Hb} & \mu_{a-730-lipid} & \mu_{a-730-water} \\ \mu_{a-760-collagen} & \mu_{a-760-HbO_2} & \mu_{a-760-Hb} & \mu_{a-760-lipid} & \mu_{a-760-water} \\ \mu_{a-800-collagen} & \mu_{a-800-HbO_2} & \mu_{a-800-Hb} & \mu_{a-800-lipid} & \mu_{a-800-water} \\ \mu_{a-850-collagen} & \mu_{a-850-HbO_2} & \mu_{a-850-Hb} & \mu_{a-850-lipid} & \mu_{a-850-water} \end{pmatrix} \bullet \begin{pmatrix} c_{collagen} \\ c_{HbO_2} \\ c_{Hb} \\ c_{lipid} \\ c_{water} \end{pmatrix}$$

By fitting the $C_{content}$ in Equation 13 to Equation 11 using a fitting method, such as a least-squares method, the constituent ratios of collagen, deoxygenated hemoglobin, and oxygenated hemoglobin can be precisely calculated, and amounts of collagen, deoxygenated hemoglobin, and oxygenated hemoglobin and their ratios to the total amount can be obtained.

As described above, after amounts of collagen, deoxygenated hemoglobin, and oxygenated hemoglobin or their ratios to the total amount for a portion on a section of the specimen E is obtained, a similar flow follows with a mapped absorption coefficient $\mu_a$ to map amounts of collagen, deoxygenated hemoglobin, and oxygenated hemoglobin or their ratios to the total amount on the section of the specimen E. By scanning this section, distributions of three-dimensional absorption-scattering information of the specimen E and the amounts of collagen, deoxygenated hemoglobin, and oxygenated hemoglobin or their ratios to the total amount can be finally obtained.

FIG. 10 illustrates mapping results of ratios of deoxygenated hemoglobin and oxygenated hemoglobin, and correlates a value of each of $C_{HbO2}$ and $C_{Hb}$ calculated at each site in the specimen with a corresponding position, expressing a degree of the value by a color depth. This expression provides quick visual confirmations of a position of a high deoxygenated hemoglobin or oxygenated hemoglobin concentration and a position of a low deoxygenated hemoglobin or oxygenated hemoglobin concentration.

When a distribution of the amounts of collagen, deoxygenated hemoglobin, and oxygenated hemoglobin or their ratios to the total amount is thus obtained, a detection precision of a tumor becomes higher than use of only the hemoglobin method or only the method for detecting a cancer with collagen.

For example, this embodiment uses a plurality of types of luminous fluxes having wavelengths of 640 nm, 730 nm, 760 nm, 800 nm, 850 nm, 910 nm and 970 nm to measure a spectroscopic characteristic of the specimen E, and thereby can precisely obtain the amounts of collagen, lipid, water, deoxygenated hemoglobin and oxygenated hemoglobin or their ratios to the total amount. When the measurement uses the above seven wavelengths, the memory 15 can store seven types of $\mu_a$ shown in Equation 14 where $\mu_{a\_\lambda}$ represents data of an absorption coefficient $\mu_a$ of a certain mapping section for each wavelength:

EQUATION 14

$$\begin{pmatrix} \mu_{a-640} \\ \mu_{a-730} \\ \mu_{a-760} \\ \mu_{a-800} \\ \mu_{a-850} \\ \mu_{a-910} \\ \mu_{a-970} \end{pmatrix}$$

$C_{content}$ as a target constituent ratio can be expressed as in Equation 15:
EQUATION 15

$$\begin{pmatrix} C_{collagen} \\ C_{HbO_2} \\ C_{Hb} \\ C_{lipid} \\ C_{water} \end{pmatrix}$$

A value of an absorption coefficient can be obtained by multiplying a distribution of an absorption coefficient $\mu_{a\_\lambda\_content}$ of each known ingredient shown in FIG. 3 by Equation 15.
EUQATION 16

$$\begin{pmatrix} \mu_{a-640-collagen} & \mu_{a-640-HbO_2} & \mu_{a-640-Hb} & \mu_{a-640-lipid} & \mu_{a-640-water} \\ \mu_{a-730-collagen} & \mu_{a-730-HbO_2} & \mu_{a-730-Hb} & \mu_{a-730-lipid} & \mu_{a-730-water} \\ \mu_{a-760-collagen} & \mu_{a-760-HbO_2} & \mu_{a-760-Hb} & \mu_{a-760-lipid} & \mu_{a-760-water} \\ \mu_{a-800-collagen} & \mu_{a-800-HbO_2} & \mu_{a-800-Hb} & \mu_{a-800-lipid} & \mu_{a-800-water} \\ \mu_{a-850-collagen} & \mu_{a-850-HbO_2} & \mu_{a-850-Hb} & \mu_{a-850-lipid} & \mu_{a-850-water} \\ \mu_{a-910-collagen} & \mu_{a-910-HbO_2} & \mu_{a-910-Hb} & \mu_{a-910-lipid} & \mu_{a-910-water} \\ \mu_{a-950-collagen} & \mu_{a-950-HbO_2} & \mu_{a-950-Hb} & \mu_{a-950-lipid} & \mu_{a-950-water} \end{pmatrix} \bullet \begin{pmatrix} C_{collagen} \\ C_{HbO_2} \\ C_{Hb} \\ C_{lipid} \\ C_{water} \end{pmatrix}$$

By fitting $C_{content}$ in Equation 16 to Equation 14 using a fitting method such as a least-squares method, the amounts of collagen, deoxygenated hemoglobin, oxygenated hemoglobin, lipid, and water or their ratios to the total amount can be precisely obtained.
As described above, after the amounts of collagen, deoxygenated hemoglobin, oxygenated hemoglobin, lipid, and water or their ratios to the total amount for a portion on a section of the specimen E is obtained, a similar flow follows with a mapped absorption coefficient $\mu_a$ so as to map an amounts of collagen, deoxygenated hemoglobin, oxygenated hemoglobin, lipid, and water or their ratios to the total amount on the section of the specimen E. By scanning this section, distributions of three-dimensional absorption-scattering information of the specimen E, and the amounts of collagen, deoxygenated hemoglobin, oxygenated hemoglobin, lipid, and water collagen or their ratios to the total amount can finally be obtained.
An acquired distribution of the amounts of collagen, deoxygenated hemoglobin, oxygenated hemoglobin, lipid, and water or their ratios to the total amount can provide a position and size of a tumor, a determination of whether the tumor is benign or malignant, and an improved detection precision of a tumor.
Whether the tissue is a normal or a tumor using deoxygenated hemoglobin and oxygenated hemoglobin is determined based on the oxygen saturation ($S_TO_2$) expressed in Equation 17, in which [X] denotes a molar concentration of one liter of X.
EQUATION 17

$$S_T O_2 = \frac{[HbO_2]}{[Hb]+[HbO_2]} \times 100[\%]$$

Conventionally, the oxygen saturation has been used to determine whether a biological tissue is a normal or a tumor, but an amount of change is small and subject to errors and the tumor determining precision is low.

In determination, this embodiment relies primarily upon the FC of collagen and the FC of lipid, and supplementally upon the oxygen saturation, and improves the determination precision between the normal and the tumor. The oxygen saturation is obtained from the amounts of deoxygenated hemoglobin and oxygenated hemoglobin for each site according to Equation 17 and used for the determination, as in the method that obtains the amounts of collagen and lipid for each site and determines a state of a biological tissue.

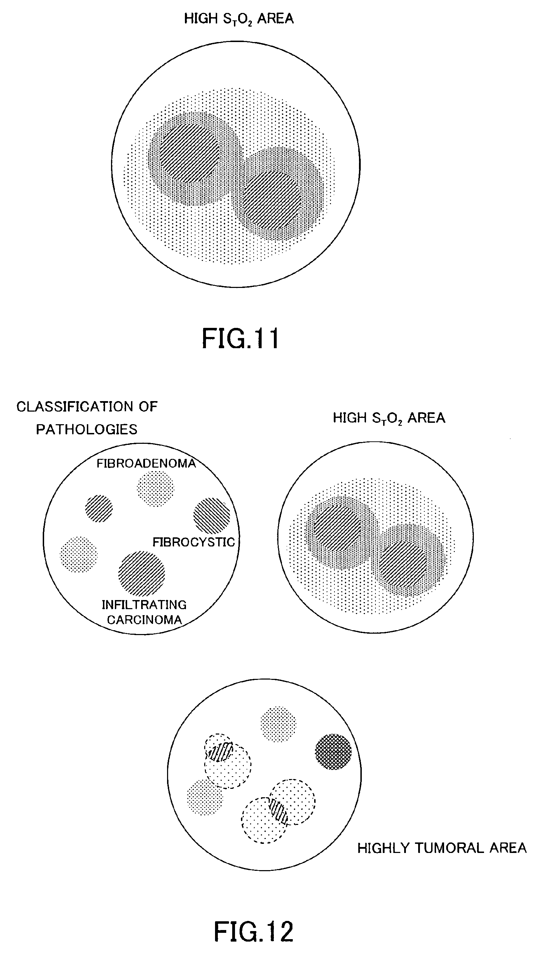

FIG. 11 is a mapping result for each value of the oxygen saturation, which is displayed by the display unit 17.

FIG. 12 is a mapping result that superposes a distribution of a state of a biological tissue onto a distribution of an oxygen saturation, which is displayed by the display unit 17. The superposition area of the two types of distributions is an area that is likely to be a tumor. This expression can precisely and easily provide a doctor and a patient with an area that is likely tumoral. The first of these two types of distributions is reliable, and may be weighed and displayed.

An amount of water, and a total amount of deoxygenated hemoglobin and oxygenated hemoglobin and the obtained scattering coefficient may be displayed on the display unit 17 and used for assistance in determining a risk of a tumor. For example, a region having a high scattering coefficient almost accords with a region having a high mammographic density which is likely tumoral. In a diagnosis, a distribution of the scattering coefficient on the display unit 17 can provide additional information.

In general, where n is the number of wavelengths, fitting is performed as shown in Equation 18.

EQUATION 18

$$\begin{pmatrix} \mu_{a\text{-}\lambda 1} \\ \mu_{a\text{-}\lambda 2} \\ \vdots \\ \vdots \\ \mu_{a\text{-}\lambda n} \end{pmatrix} \leftarrow \begin{pmatrix} \mu_{a\text{-}\lambda 1\text{-}water} & \mu_{a\text{-}\lambda 1\text{-}lipid} & \mu_{a\text{-}\lambda 1\text{-}Hb} & \mu_{a\text{-}\lambda 1\text{-}HbQ} & \mu_{a\text{-}\lambda 1\text{-}collagen} \\ \mu_{a\text{-}\lambda 2\text{-}water} & \mu_{a\text{-}\lambda 2\text{-}lipid} & \mu_{a\text{-}\lambda 2\text{-}Hb} & \mu_{a\text{-}\lambda 2\text{-}HbQ} & \mu_{a\text{-}\lambda 2\text{-}collagen} \\ \vdots & \vdots & \vdots & \vdots & \vdots \\ \vdots & \vdots & \vdots & \vdots & \vdots \\ \mu_{a\text{-}\lambda n\text{-}water} & \mu_{a\text{-}\lambda n\text{-}lipid} & \mu_{a\text{-}\lambda n\text{-}Hb} & \mu_{a\text{-}\lambda n\text{-}HbQ} & \mu_{a\text{-}\lambda n\text{-}collagen} \end{pmatrix} \bullet \begin{pmatrix} C_{water} \\ C_{lipid} \\ C_{Hb} \\ C_{HbQ} \\ C_{collage} \end{pmatrix}$$

As described above, after constituent ratios of one portion on one section in the specimen E are obtained, a similar flow follows with a mapped absorption coefficient $\mu_a$ so as to map the constituent ratios on the section of the specimen E. By scanning this section, distributions of three-dimensional absorption-scattering information of the specimen E, and an amount of collagen or a ratio of collagen to the total amount can be finally obtained.

Instead of modulating the light source 2, a short pulse of a few picoseconds may be introduced into the specimen E, and the $\mu_a$ and $\mu_s'$ may be estimated based on an output time waveform. The aforementioned method may be used to estimate the ingredient based on obtained $\mu_a$ and $\mu_s'$. This method can also provide the distributions the three-dimensional absorption-scattering information of the specimen E and the ingredients. Alternatively, it is possible to irradiate the light having a constant intensity into the specimen E, to find $\mu_a$ from the transmitted light intensity, and to estimate the ingredient from obtained $\mu_a$.

SECOND EMBODIMENT

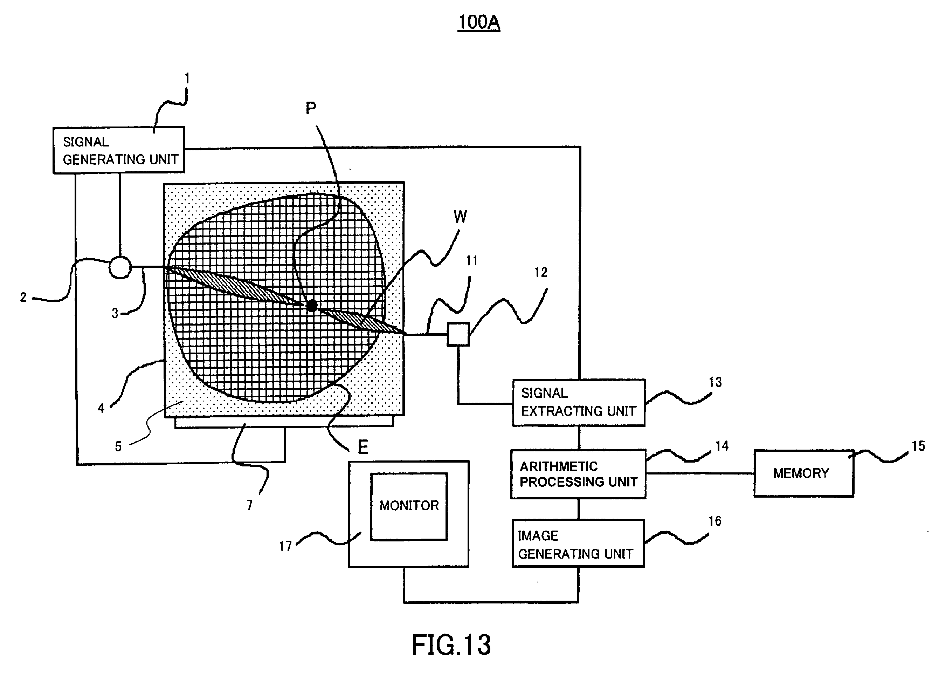

FIG. 13 is a block diagram of a measurement apparatus 100A according to a second invention of the present invention. The measurement apparatus 100A measures a spectroscopic characteristic of a specimen E using AOT. Those elements in FIG. 13, which are the same as corresponding elements in FIG. 1, are designated by the same reference numerals. The measurement apparatus 100A can also be used instead of the measurement apparatus 100 shown in the FIG. 2.

The coherent light such as a laser beam is continuously emitted from the light source 2. The emitted light enters a side of the measurement vessel 4 through the optical fiber 3. The light entering the vessel propagates while repeating absorptions and scatters in the medium.

An ultrasound transducer array 7 arranged on the bottom of the measurement vessel 4 is driven using a sine wave signal of f with the signal generating unit 1. The ultrasound transducer array 7 is controlled to irradiate the ultrasound so that the sound pressure is focused onto the measurement site as a local region in the measurement vessel 4. The ultrasound focusing area P changes a density of the medium due to the sound pressure, and changes the refractive index and the scattering coefficient of the medium.

When the light passes the ultrasound focusing area P, its phase is modulated by the changes of the refractive index and the scattering coefficient of the medium. The light modulated in the ultrasound focusing area P propagates in the medium as light modulated by the drive frequency f of the ultrasound.

The light detecting device 12 receives the light modulated by the ultrasound and non-modulated light, and detects signals from both modulated light and non-modulated light.

A signal extracting unit 13 performs a Fourier transformation for the detected signals to separate a non-modulated signal I1 and a signal I2 modulated by ultrasound frequency f. The separated signal and a reference signal are used to calculate an absorption coefficient and a scattering coefficient in the specimen. Similarly, the arithmetic processing unit 14 calculates ratios of the ingredients of the specimen E based on the absorption coefficient and the scattering coefficient, and the memory 15 stores the calculated absorption coefficient and scattering coefficient and ratios of the ingredients. An image-generating unit 16 maps the stored values, and the display unit 17 displays the distributions of three-dimensional absorption coefficient and scattering coefficient and the ratios of the ingredients.

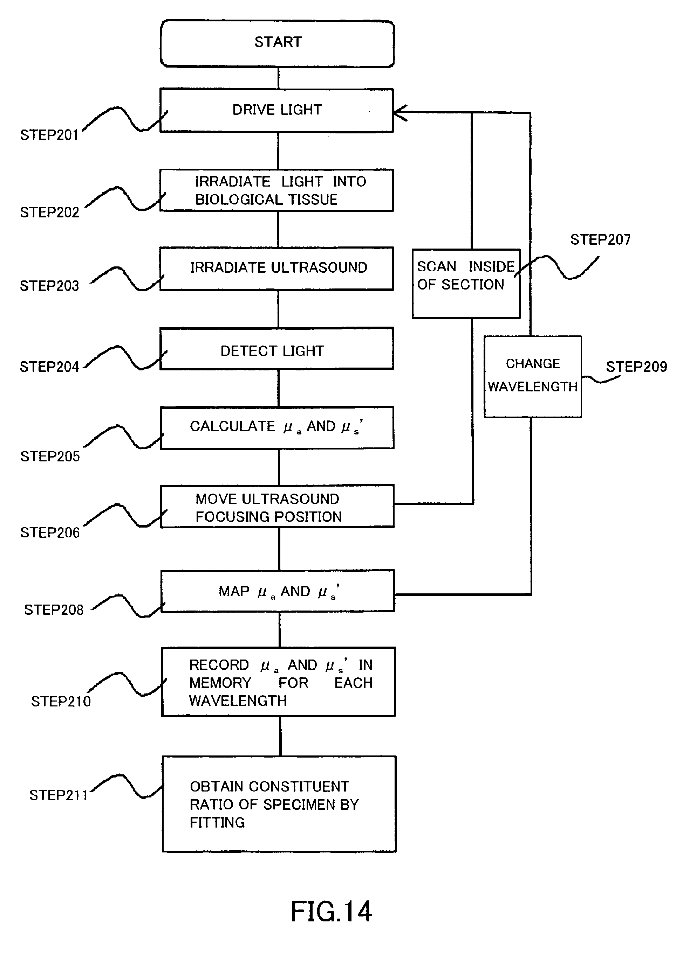

FIG. 14 is a flowchart for explaining an operation of the measurement apparatus 100A to obtain a tomographic image on one section of the specimen E. Initially, in the step 201, the signal generating unit 1 drives the light source 2. In the step 202, the light source 2 introduces the light into the specimen E through the optical fiber 3. In the step 203, the ultrasound transducer array 7 irradiates and focuses ultrasound into the specimen E. In the step 204, the light detecting device 12 detects the light. In the step 205, the absorption coefficient $\mu_a$ and the equivalent scattering coefficient $\mu_s'$ are calculated based in the light intensity detected by the light detecting device 12 using Equations 2 and 3.

Next, in the step 206, the ultrasound transducer array 7 is controlled to shift a focusing position of the sound pressure. Next, the step 207 arranges local regions (ultrasound focusing areas P) tagged by an interaction between the light and the ultrasound throughout the whole region of the absorption-scattering body in the measurement vessel 4. The absorption coefficient and scattering coefficient of the local region tagged by the interaction between the light and the ultrasound can be obtained by recursively finding a difference of the known region and the unknown region in the specimen E. The step 208 maps these values, and easily provides a tomographic view of one section of specimen E. Here, the mapping can be performed by correlating these values to the section of the specimen and displaying the sectional view by color in each range of these values, which is divided into a plurality of ranges, based on these values or an area to which these values belong.

Next, the step 209 changes a wavelength of the driven light source. For this method, a plurality of light sources having different wavelengths may introduce the luminous fluxes from separate locations or from bundled fibers at one location. Alternatively, a white light source may be prepared, and the light from the white light may be separated by a diffraction grating, or the light having only a specific wavelength may be selected by a wavelength filter. The modulated light having another wavelength is introduced into the specimen E by any one of these methods, and the operation of the above steps 201-207 is repeated, and the memory 15 stores calculated $\mu_a$ and $\mu_s'$ for each wavelength in the step 210. As in the first embodiment, the number of used wavelengths is more than the number of ingredients of the specimen E.

Similar to the first embodiment, the wavelength of the incident light is determined by the ingredient to be detected. A wavelength used for the measurement and means for finding the ingredient are as described in the first embodiment. This embodiment provides the constituent ratios of water, lipid, oxygenated hemoglobin, deoxygenated hemoglobin, and collagen of the local region tagged by the interaction between the light and the ultrasound. By scanning this section, the three-dimensional absorption-scattering information of the specimen E (step 210) and the distribution of the ingredients (step 211) can be finally obtained.

THIRD EMBODIMENT

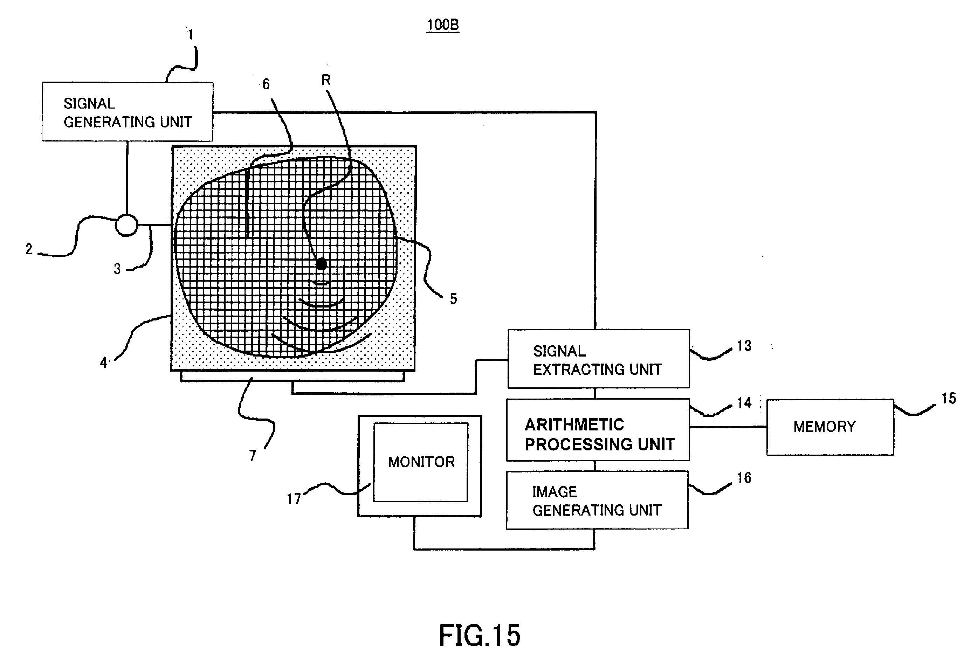

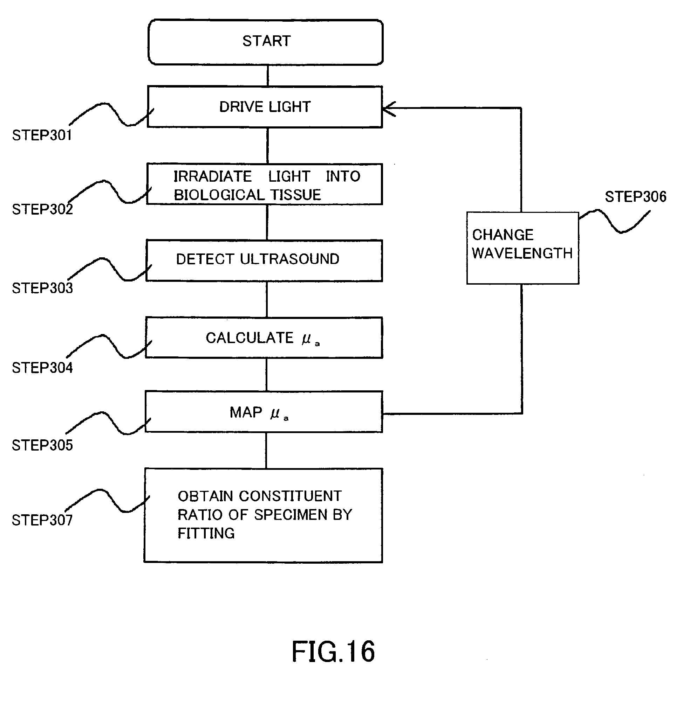

FIG. 15 is block diagram of a measurement apparatus 100B according to a third embodiment of the present invention. The measurement apparatus 100B measures a spectroscopic characteristic of a specimen E using PAT. Those elements in FIG. 16, which are the same as corresponding elements in FIG. 1, are designated by the same reference numerals. The measurement apparatus 100B can also be used instead of the measurement apparatus 100 shown in the FIG. 2.

The light source 2 using a semiconductor laser emits the pulsed light of a nanosecond order via the signal generating unit 1. The pulsed light from light source 2 enters a side of the measurement vessel 4 through the optical fiber 3. The light introduced into the vessel propagates in the specimen E and causes, when the propagating light reaches an absorber (measurement site) R, an elastic wave due to the expansion and contraction of the medium. The elastic wave from the absorber R propagates in the specimen E, and is detected by the ultrasound transducer array 7.

The signal extracting unit 13 maintains a synchronization of the signal generating unit 1, and a position of the absorber R can be found based on a difference of time detected by each array. An absorption coefficient $\mu_a$ of the absorber R can be calculated based on the intensity of the elastic wave. The arithmetic processing unit 14 calculates the ratios of the ingredients in the specimen E based on the absorption coefficient, and the memory 15 stores the calculated absorption coefficient and scattering coefficient and the ratio of the ingredients. The image-generating unit 16 maps stored values, and the display unit 17 displays the three-dimensional absorption coefficient and scattering coefficient and distribution of the ratios of the ingredients.

FIG. 16 is a flowchart for explaining an operation of the measurement apparatus 100B to acquire a tomographic view of one section of the specimen E. Initially, the step 301 drives light source 2, and the step 302 introduces the nanosecond order light into the specimen E. The step 303 detects the ultrasound from the absorber R. The step 304 calculates $\mu_a$ based on the intensity, and the step 305 calculates a position based on a time difference and maps it. The step 306 changes a wavelength similar to the step 209, and the step 307 finds a constituent ratio of the specimen E through fitting. The memory 15 stores calculated $\mu_a$ and $\mu_s$' and the constituent ratio. Similar to the first embodiment, the number of used wavelengths is more than the number of ingredients of the specimen E.

The wavelength used for the measurement and a method for finding the ingredient based on it are the same as those in the first embodiment. This embodiment can provide the constituent ratios of water, lipid, oxygenated hemoglobin, deoxygenated hemoglobin, and collagen of the local region. By scanning this section, the distributions of the three-dimensional absorption-scattering information and the ingredients of the specimen E can be finally obtained.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A measurement apparatus (100) comprising:

   a measurement means which measures the spectroscopic characteristics of the inside of a specimen (E) by irradiating a plurality of types of light, each having a different wavelength within the wavelength range of 600nm to 1,000nm, on the specimen;

   **characterized in that** said measurement apparatus further comprises:

   an arithmetic processing means (14) which calculates the ratio of both collagen and lipid relative to the whole of a plurality of ingredients including collagen and lipid from a measurement result of the measurement means and the absorption coefficients ($\mu_a$) of each ingredient, and determines the relationship of the fitting coefficients of the lipid and collagen and the state of biological tissue, and then determines the state of biological tissue of the specimen from the ratio of collagen and the ratio of lipid which were calculated; and
   a display unit (17) which displays a result of processing by the arithmetic processing means,
   wherein the measurement means uses a light having a predetermined wavelength within the wavelength range between 600nm and 700nm and at least two types of light having different wavelengths within the wavelength range between 730nm to 760nm as the plurality of types of light.

2. The measurement apparatus according to claim 1, wherein the plurality of ingredients further contains water; and wherein the measurement means uses two types of light having different wavelengths within the wavelength range between 900 nm and 1000 nm as the plurality of types of light, and the arithmetic processing means calculates at least one of the following: the quantities of lipid and water, the ratio relative to the whole of the plurality of ingredients, and the distribution of the total quantities of lipid and water.

3. The measurement apparatus according to any one of claim 1 or 2, wherein the arithmetic processing means calculates at least one of the following: the quantities of collagen and lipid, the ratio relative to the whole of the plurality of ingredients, and the distribution of the total quantities of collagen and lipid.

4. The measurement apparatus according to any one of claims 1 to 3, wherein the plurality of ingredients further contains reduced hemoglobin and oxygenated hemoglobin; and wherein the measurement means uses at least two types of light having different wavelengths within the wavelength range between 760nm and 850nm as the plurality of types of light; and the arithmetic processing means calculates at least one of the following from a measurement result of the measurement means: the quantities of reduced hemoglobin and oxygenated hemoglobin, the ratio relative to the whole of the plurality of ingredients, and the total quantities of reduced hemoglobin and oxygenated hemoglobin.

5. The measurement apparatus according to claim 4, wherein the arithmetic processing means calculates oxygen saturation ($S_TO_2$) of hemoglobin, given by the ratio of the molar concentration of oxygenated hemoglobin relative to the total molar concentrations of reduced hemoglobin and oxygenated hemoglobin, at each location.

6. The measurement apparatus according to claim 6, wherein the display unit displays the distribution of the state and the distribution of the oxygen saturation of the hemoglobin by superposing them.

7. The measurement apparatus according to any one of claims 1 to 6, wherein the measurement means has a light detecting device (12) for detecting diffused light that is diffused from the specimen, and the arithmetic processing means calculates the absorption coefficient of the specimen and calculates the ratio of each of the ingredients of the specimen from the absorption coefficient of the specimen.

8. The measurement apparatus according to any one of claims 1 to 6, wherein the measurement means has an ultrasound transducer array (7) for generating ultrasound and concentrating it on a specimen and a light detecting device for detecting a signal generated by the mutual interactions of incident light in the area to be tested and the concentrated ultrasound, and the arithmetic processing means the calculates absorption coefficient of the specimen from the signal and calculates the ratio of each of the ingredients of the specimen from the absorption coefficient of the specimen.

9. The measurement apparatus according to claims 1 to 6, wherein the measurement means has an ultrasound transducer array for detecting elastic waves from an area to be tested inside the specimen, and the arithmetic processing means calculates the position and absorption coefficient of the area to be tested and calculates the ratio of each of the ingredients of the specimen from the absorption coefficient of the specimen.

EP 2 036 489 A2

FIG.1

FIG.2

START

STEP101 — DRIVE LIGHT

STEP102 — IRRADIATE LIGHT INTO BIOLOGICAL TISSUE

STEP103 — DETECT LIGHT

STEP104 — CALCULATE $\mu_a$ and $\mu_s'$

STEP105 — MOVE LIGHT SOURCE POSITION BY $\Delta d$

STEP106 — SCAN INSIDE OF SECTION

STEP108 — CHANGE WAVELENGTH

STEP107 — MAP $\mu_a$ AND $\mu_s'$

STEP109 — RECORD $\mu_a$ AND $\mu_s'$ IN MEMORY FOR EACH WAVELENGTH

STEP110 — OBTAIN CONSTITUENT RATIO OF SPECIMEN BY FITTING

FIG.3

FIG.4

FIG.5

DISTRIBUTION OF COLLAGEN

FIG.6

DISTRIBUTION OF LIPID

DISTRIBUTION OF WATER

FIG.7

FIG.8

EP 2 036 489 A2

CLASSIFICATION OF PATHOLOGIES

FIBROADENOMA

FIBROCYSTIC

INFILTRATING
CARCINOMA

FIG.9

DISTRIBUTION OF Hb

DISTRIBUTION OF $HbO_2$

FIG.10

22

HIGH S$_T$O$_2$ AREA

# FIG.11

CLASSIFICATION OF
PATHOLOGIES

HIGH S$_T$O$_2$ AREA

FIBROADENOMA

FIBROCYSTIC

INFILTRATING
CARCINOMA

HIGHLY TUMORAL AREA

# FIG.12

100A

FIG.13

SIGNAL
GENERATING UNIT

MONITOR

MEMORY

SIGNAL
EXTRACTING UNIT

ARITHMETIC
PROCESSING UNIT

IMAGE
GENERATING UNIT

START

STEP201 — DRIVE LIGHT

STEP202 — IRRADIATE LIGHT INTO BIOLOGICAL TISSUE

STEP203 — IRRADIATE ULTRASOUND

STEP207 — SCAN INSIDE OF SECTION

STEP204 — DETECT LIGHT

STEP209 — CHANGE WAVELENGTH

STEP205 — CALCULATE $\mu_a$ AND $\mu_s'$

STEP206 — MOVE ULTRASOUND FOCUSING POSITION

STEP208 — MAP $\mu_a$ AND $\mu_s'$

STEP210 — RECORD $\mu_a$ AND $\mu_s'$ IN MEMORY FOR EACH WAVELENGTH

STEP211 — OBTAIN CONSTITUENT RATIO OF SPECIMEN BY FITTING

FIG.14

FIG.15

START

STEP301 — DRIVE LIGHT

STEP302 — IRRADIATE LIGHT INTO BIOLOGICAL TISSUE

STEP303 — DETECT ULTRASOUND

STEP304 — CALCULATE $\mu_a$

STEP305 — MAP $\mu_a$

STEP306 — CHANGE WAVELENGTH

STEP307 — OBTAIN CONSTITUENT RATIO OF SPECIMEN BY FITTING

FIG.16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3107914 B

**Non-patent literature cited in the description**

- **MIT, HAKA.** Diagnosing breast cancer by using Raman spectroscopy. *Proc Natl Acad Sci USA*, 2005

- Absorption properties of breast: the contribution of collagen. *ULTRAS-CNR-INFM and IFN-CNR*, 2006